# EUROPÄISCHE PATENTSCHRIFT

(11) **EP 4 474 647 B1**
(45) Veröffentlichungstag und Bekanntmachung des Hinweises auf die Patenterteilung: **25.06.2025**
(21) Anmeldenummer: 24179554.1
(22) Anmeldetag: 03.06.2024
(51) Int. Cl.: F04B 43/12, A61M 60/279, A61M 60/113, A61M 60/37, A61M 60/441

(54) **PERISTALTIKPUMPE**
PERISTALTIC PUMP
POMPE PÉRISTALTIQUE

(30) Priorität: 07.06.2023 DE 102023115053
(43) Veröffentlichungstag der Anmeldung: 11.12.2024
(73) Patentinhaber: B. Braun Avitum AG, 34212 Melsungen (DE)
(72) Erfinder: JAKOBI, Marco, 37318 Uder (DE)
(74) Vertreter: Winter, Brandl - Partnerschaft mbB

(56) Entgegenhaltungen:
- EP-B1- 1 749 549
- US-A1- 2023 041 478
- US-B2- 8 568 115

## Beschreibung

Die Erfindung betrifft ein eine Schlauchrollen- bzw. Peristaltikpumpe, d.h. eine Verdrängerpumpe, bei der das zu fördernde Medium durch äußere mechanische Verformung eines Schlauches durch diesen hindurchgedrückt wird, gemäß dem Oberbegriff des Patentanspruchs 1. Derartige Pumpen werden häufig zum Fördern von Fluid, insbesondere Blut, in einer Vorrichtung zur extrakorporalen Blutbehandlung, insbesondere in einer Dialysemaschine, verwendet. Das Fluid wird dabei mittels der Peristaltikpumpe von einer Niederdruckseite zu einer Hochdruckseite gefördert, wobei eine elastisch verformbare und zwischen der Niederdruckseite und der Hochdruckseite angeordnete Fluidleitung in Form eines Schlauchsegments, welches als Pumpensegment bezeichnet wird, zwischen einer Stützfläche eines Pumpenbetts und einem gegenüber dieser rotierenden Rotor mit zumindest zwei Quetschelementen verformt, insbesondere zusammengequetscht.

Bei einer bekannten Peristaltikpumpe, wie sie beispielsweise aus dem Dokument EP 1 749 549 B1 bekannt und ist, sind - wie in Figur 7 bis 9 schematisch gezeigt - am Rotor 310 mit der Drehachse A zwei diametral zueinander versetzt angeordnete Quetschelemente 320 in Form von gefedert gelagerten Andruckrollen vorgesehen, und die Stützfläche 330 wird von einer Kreissegmentfläche gebildet, die sich über einen ausreichend großen Zentriwinkel größer als 180° erstreckt.

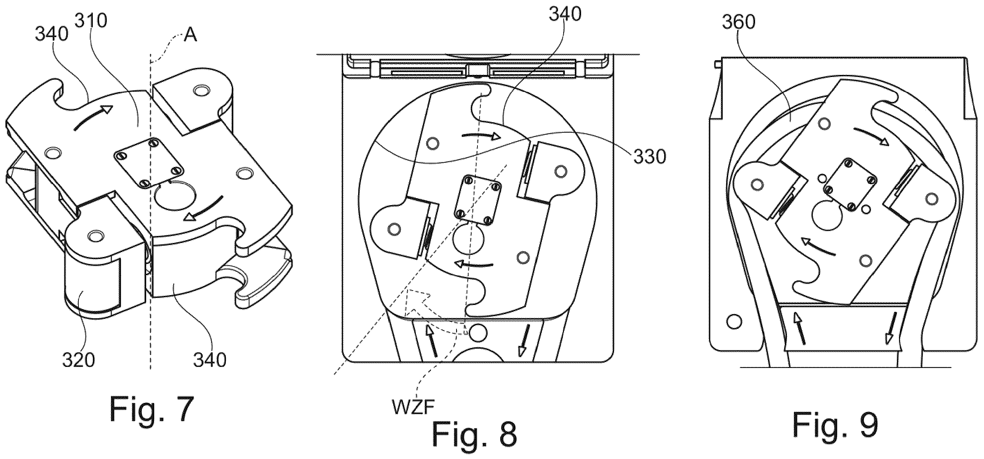

Um den Schlauch, also ein in der Peristaltikpumpe eingelegtes Pumpensegment 360 (siehe Figur 9) im Betrieb einigermaßen zu fixieren, befindet sich in der Mitte zwischen den beiden Andruckrollen 320 jeweils eine kreissegmentartig ausgeführte Führungsfläche 340, die sich über einen Zentriwinkel WZF von etwa 30° erstreckt. Es zeigt sich, dass durch diese Anordnung das Handling beim manuellen Einlegen des Pumpensegmentes erschwert wird.

Der Erfindung liegt daher die Aufgabe zugrunde, eine gattungsgemäße Peristaltikpumpe derart weiterzubilden, dass das Handling beim manuellen Einlegen des Pumpensegments vereinfacht wird.

Diese Aufgabe wird mit den Merkmalen des Patentanspruchs 1 gelöst.

Die Erfindung beruht auf der Erkenntnis, dass sich dann, wenn keine besonderen Maßnahmen zur Fixierung des Pumpensegments ergriffen werden, das Pumpensegment während des Betriebs der Peristaltikpumpe entgegen der Förderrichtung im Pumpenbett bewegt, wodurch sich das Pumpensegment - wie in Figur 9 dargestellt - immer enger um den Rotor zieht. Hierdurch kann es zu Beschädigungen am Schlauch und zu Schwankungen des Fördervolumens kommen.

Zur Lösung der Aufgabe werden mit dem Rotor mitdrehende Führungsflächen vorgesehen, die jeweils zu beiden Seiten des Quetschelements, also paarweise in Umfangsrichtung benachbart derart angeordnet und gestaltet sind, dass sie mit der Stützfläche jeweils einen kreissegmentartigen Führungskanal ausbilden, in dem das Schlauchsegment mit vorgegebener Spielpassung radial fixierbar ist. Hierdurch ergibt sich der zusätzliche Vorteil, dass sich dadurch nicht nur das Fördervolumen der Pumpe konstant halten lässt, sondern auch das Pumpensegment im Betrieb besonders schonend ,behandelt' wird. Über den Radius der Führungsflächen wird das Krümmungsverhalten des Schlauches definiert, wodurch das Fördervolumen konstant gehalten wird. Gleichzeitig verhindern die Führungsflächen eine Beschädigung des Pumpensegmentes. Weil die Führungsflächen nur in der Nachbarschaft der Quetschelemente ausgebildet sind, wird die Führungskontur in mehrere kleine Bereiche aufgeteilt, wodurch die Kontaktfläche verringert wird, was ebenfalls positiv auf den Abrieb am Schlauch auswirkt.

Wenn die einem Quetschelement zugeordneten und diesem benachbarten Führungsflächen jeweils Teilsegmente eines Kreises beschreiben, ergeben sich im Zusammenwirken mit einer zylindrischen bzw. torusähnlichen Stützfläche zu beiden Seiten des Quetschelements kreisförmig gebogene Führungskanäle, mit denen das Krümmungsverhalten des Schlauchs noch besser kontrolliert werden kann.

Wenn sich die Führungsflächen über einen Gesamt-Zentriwinkel erstrecken, der so bemessen ist, dass zwischen Führungsflächen benachbarter Quetschelemente ein sich in Umfangsrichtung erstreckender Freiraum belassen wird, ergibt sich der zusätzliche Vorteil, dass dieser Freiraum für eine manuelle seitliche Entriegelung der Rotorabdeckung bzw. des Rotors und beim Einlegen des Schlauch- bzw. Pumpensegments genutzt werden kann.

Gemäß einer vorteilhaften Weiterbildung erstrecken sich die einem Quetschelement zugeordneten Führungsflächen über unterschiedlich große Zentriwinkel. Vorzugsweise ist der Zentriwinkel der dem Quetschelement nachlaufenden Führungsfläche größer, wodurch sich die Schlauchkrümmung bei möglichst geringem Materialverbrauch noch besser stabilisieren lässt.

Grundsätzlich können die Führungsflächen an beliebigen, sich mit dem Rotor mitbewegenden Komponenten angebracht sein. Besondere zusätzliche Vorteile ergeben sich dann, wenn die Führungsflächen an einer Rotorabdeckung ausgebildet oder an dieser optional auswechselbar befestigt sind. Denn dadurch ergibt sich eine besonders große Gestaltungsfreiheit bei gleichzeig einfacher Herstellbarkeit, weil derartige Rotorabdeckungen häufig als Kunststoff-Spritzgussteile ausgebildet sind.

Es ist grundsätzlich möglich, die Erfindung auch bei Peristaltikpumpen einzusetzen, bei denen die Quetschelemente in veränderbarem Winkelabstand zueinander liegen. Wenn die Quetschelemente allerdings um 180° zueinander versetzt angeordnet sind, ist es von Vorteil, jeweils zwei Führungsflächen rotationssymmetrisch zueinander auszubilden.

Vorteilhafterweise sind die Führungsflächen von einem Werkstoff gebildet und/oder beschichtet, der abriebminimierende Gleiteigenschaften hat. Darüber hinaus ist es von Vorteil, wenn die Oberflächen der Führungsflächen mit hoher Oberflächenqualität ausgestattet werden.

Nachstehend werden beispielhafte Ausführungformen der Erfindung näher erläutert. Es zeigen:
Figur 1 eine perspektivische Ansicht der Peristaltikpumpe mit geöffnetem Gehäusedeckel;
Figur 2 eine perspektivische Ansicht eines Rotors der Peristaltikpumpe gemäß Figur 1;
Figur 3 eine Draufsicht der Peristaltikpumpe;
Figur 4 eine schematische Draufsicht der Peristaltikpumpe mit eigelegtem Pumpensegment;
Figur 5 eine der Figur 4 ähnliche schematische Draufsicht der Peristaltikpumpe mit freigeschnittener Rotorabdeckung im Bereich der Quetschelemente;
Figur 6 eine Draufsicht der Peristaltikpumpe mit abgenommener Rotorabdeckung;
Figur 7 eine perspektivische Ansicht eines Rotors einer bekannten Peristaltikpumpe;
Figur 8 eine schematische Draufsicht der bekannten Peristaltikpumpe mit eingesetztem Rotor; und
Figur 9 die Draufsicht der bekannten Peristaltikpumpe mit eingelegtem Pumpensegment.

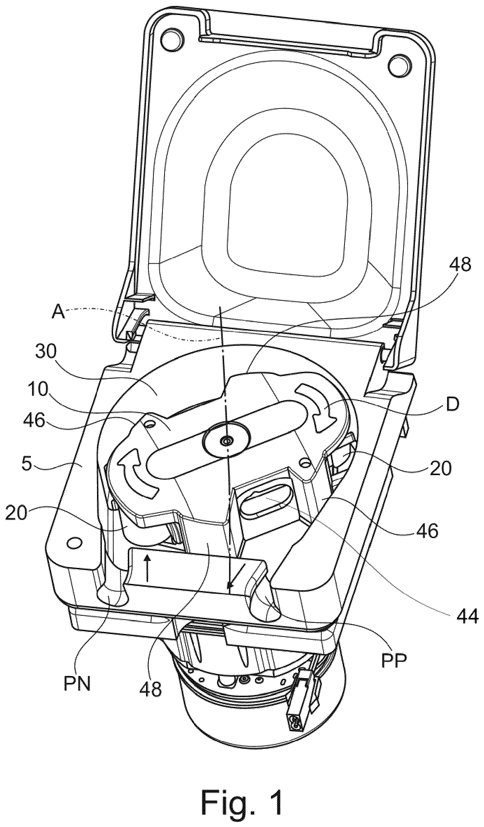

In Figur 1 ist mit dem Bezugszeichen 1 eine Peristaltikpumpe bezeichnet, wie sie beispielsweise in Dialysemaschinen eingesetzt wird. Die Peristaltikpumpe hat dort die Aufgabe, ein definiertes Volumen eines Mediums, wie zum Beispiel Blut oder Dialysefluid, durch Verformen und Abklemmen der elastisch verformbaren Fluidleitung zu fördern. Die Peristaltikpumpe zur Förderung von Blut fördert in der Regel von einer negativen Druckseite PN (Niederdruckseite) auf eine positive Druckseite PP (Hochdruckseite). Die Peristaltikpumpe hat ein Pumpengehäuse 5, in dem ein um eine Rotorachse A rotierbarer Rotor 10 mit zumindest zwei in Umfangsrichtung zueinander beispielsweise diametral versetzten Quetschelementen 20 , hier Druckrollen, aufgenommen ist und welches eine sich bogenförmig um die Rotorachse A erstreckende und radial von dem Rotor 20 beabstandete Stützfläche 30 aufweist, die zum Abstützen eines radial zwischen den Rotor 20 und die Stützfläche 30 einbringbaren, in Figur 1 nicht gezeigten Schlauchsegments eingerichtet ist. Die Stützfläche 30 ist in der Regel von einer Zylinderfläche gebildet, sie kann jedoch auch muldenförmig vertieft sein. Die Drehrichtung des Rotors 10 beim Fördern von Fluid ist mit dem Pfeil D bezeichnet.

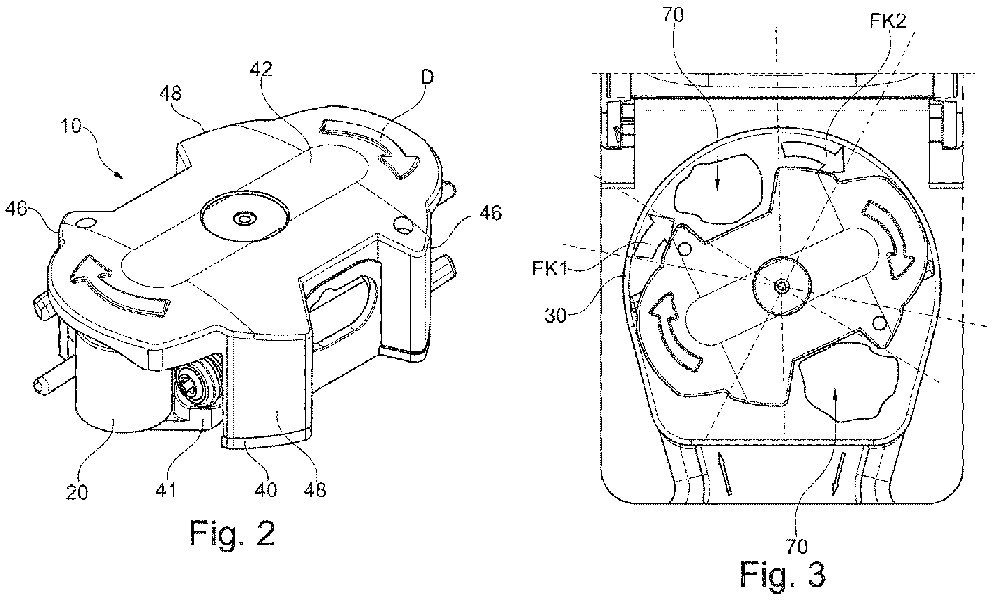

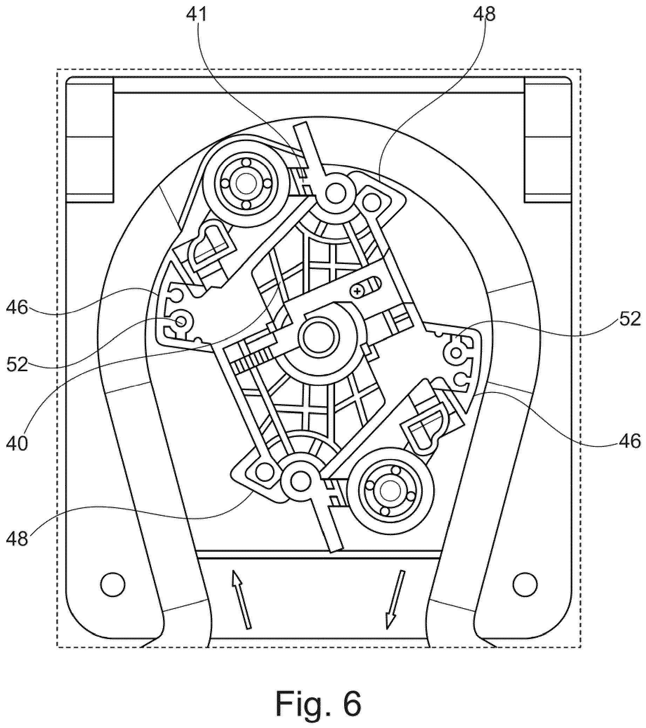

Figur 2 zeigt Einzelheiten des Rotors 20. Er hat einen in Figur 6 in Draufsicht sichtbaren Rotor-Grundkörper 40 und eine Rotorabdeckung 42. Der Rotor 20 ist durch eine seitlich bedienbare Taste 44 von einer nicht näher gezeigten Antriebswelle abnehmbar. Die Quetschelemente 20 sind drehbar auf einer gelenkig am Rotor-Grundkörper 40 federnd abgestützten Schwinge 41 gelagert.

Die sich mit dem Rotor 10 mitdrehende Rotorabdeckung 42 trägt im Bereich der Quetschelemente 20 Führungsflächen 46, 48, von denen die eine Führungsfläche 46 dem Quetschelement 20 vorläuft und die andere Führungsfläche 48 dem Quetschelement 20 nachläuft. Die Führungsflächen 46, 48 sind demnach jeweils zu beiden Seiten des Quetschelements 20 in Umfangsrichtung benachbart derart angeordnet und gestaltet, dass sie - wie aus den Figuren 3 bis 6 ersichtlich - mit der Stützfläche 30 jeweils einen kreissegmentartigen Führungskanal FK1 und FK2 ausbilden, in dem das in das Pumpengehäuse 1 eingelegte Schlauchsegment 60, das auch als Pumpensegment bezeichnet wird, mit vorgegebener Spielpassung radial fixierbar ist.

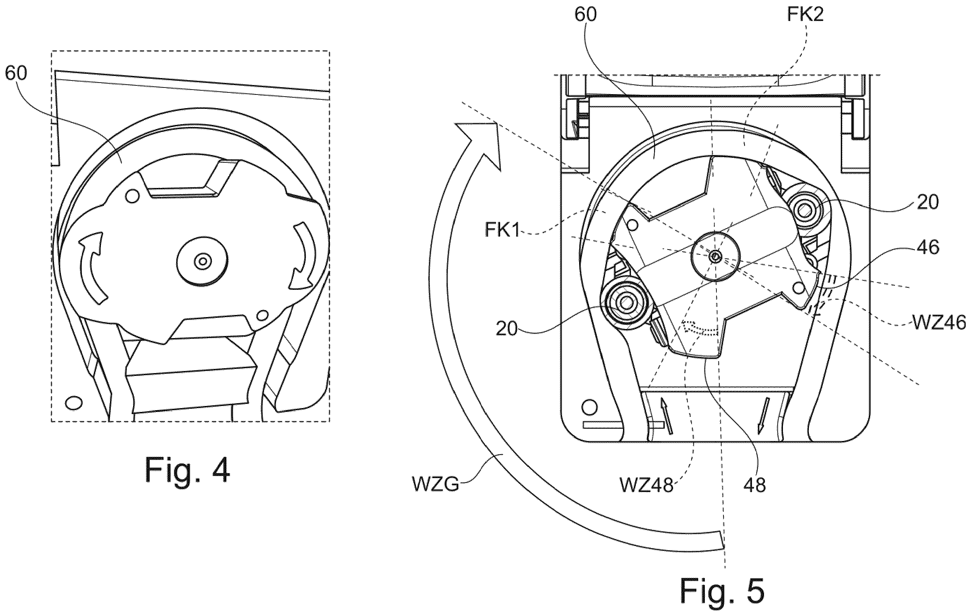

Über den Radius der Führungsflächen wird auf diese Weise - wie den Darstellungen der Figuren 4 und 5 entnommen werden kann - das Krümmungsverhalten des Schlauches 60 im Betrieb der Peristaltikpumpe definiert. Somit kann das Fördervolumen konstant gehalten werden. Gleichzeitig verhindern die Führungsflächen eine Beschädigung des Pumpensegments 60. Vorzugsweise sind die Führungsflächen 46, 48 von einem Werkstoff gebildet und/oder beschichtet, der abriebminimierende Gleiteigenschaften hat.

Die Anordnung der Führungsflächen 46, 48 ist - wie aus den Figuren ersichtlich - demnach derart, dass sie nur in der Nachbarschaft der Quetschelemente 20 ausgebildet sind, also in einem eingeschränkten Winkelabstand von etwa 20° bis 40° für die vorlaufende Führungsfläche 46 bzw. von 35° bis 60° für die nachlaufende Führungsfläche 48. Auf diese Weise wird die Führungskontur in mehrere die Führungskanäle FK1 und FK2 bestimmende kleine Bereiche aufgeteilt, wodurch die Kontaktfläche verringert wird, was sich ebenfalls positiv auf den Abrieb am Schlauch 60 auswirkt. Mit dieser Anordnung erstrecken sich die Führungsflächen 46, 48 über einen Gesamt-Zentriwinkel WZG (siehe Figur 5), der beispielsweise mit einer Beschränkung auf 110° bis 140° so bemessen ist, dass zwischen benachbarten Führungsflächen 46 und 48 ein sich in Umfangsrichtung erstreckender Freiraum 70 (siehe Figur 3) belassen wird. Dieser Freiraum 70 kann in vorteilhafter Weise für eine manuelle seitliche Entriegelung des Rotors 10 und beim Einlegen des Schlauch- bzw. Pumpensegments 60 genutzt werden.

Im gezeigten Ausführungsbeispiel beschreiben die einem Quetschelement 20 zugeordneten und diesem benachbarten Führungsflächen 46, 48 jeweils Teilsegmente eines Kreises. Hierdurch ergeben sich - wie in Figur 5 dargestellt - im Zusammenwirken mit einer zylindrischen bzw. torusähnlichen Stützfläche 30 zwischen den Quetschelementen 20 kreisförmig gebogene Führungskanäle, mit denen das Krümmungsverhalten des Schlauchs 60 besonders wirksam kontrolliert werden kann.

Für die Gestaltung der Führungsflächen 46, 48 verbleibt ein großer Spielraum hinsichtlich Lage, Form und Größe. Beim gezeigten Ausführungsbeispiel erstrecken sich die einem Quetschelement 20 zugeordneten Führungsflächen 46, 48 über unterschiedlich große Zentriwinkel, wobei sich die dem Quetschelement 20 nachlaufende Führungsfläche 48 über einen größeren Zentriwinkel WZ48 erstreckt als die dem Quetschelement 20 vorlaufende Führungsfläche 46 (Figur 5).

Da bei der gezeigten Peristaltikpumpe die Quetschelemente 20 diametral zueinander versetzt angeordnet sind, sind die vorlaufenden und nachlaufenden Führungsflächen 46 und 48 rotationssymmetrisch zueinander ausgebildet.

Grundsätzlich können die Führungsflächen 46, 48 an jeder beliebigen, sich mit dem Rotor 10 mitbewegten Komponente ausgebildet sein. Im gezeigten Ausführungsbeispiel sind sie an der Rotorabdeckung 42 ausgebildet, die lösbar an einem Rotor-Grundkörper 50 angebracht ist. Der Rotor-Grundkörper 50 ist in Figur 6 in Draufsicht bei abgenommener Rotorabdeckung 42 gezeigt. Man erkennt, dass ein Sockel 52 des Rotor-Grundkörpers 50 bereits die Kontur der Führungsflächen 46, 48 hat.

Selbstverständlich sind Abweichungen von dem gezeigten Ausführungsbeispiel möglich, ohne den Grundgedanken der Erfindung zu verlassen.

So können die Quetschelemente 20 beispielsweis auch in Rotationsrichtung relativ zueinander winkelpositionierbar ausgebildet sein.

Eine die Führungsflächen 46, 48 ausbildende Komponente der Peristaltikpumpe kann auch einstückig mit dem Rotor-Grundkörper 40 ausgebildet oder auch auswechselbar am Rotor-Grundkörper 40 oder an der Rotorabdeckung 42 angebracht sein. Dabei kann die Rotorabdeckung 42 mehrteilig, beispielsweise als 2K-Teil ausgebildet sein, wobei in diesem Fall die Führungsflächen 46, 48 an Einlegeteilen ausgebildet sind.

Es liegt auch im Bereich der Erfindung, wenn der Rotor mehr als zwei Quetschelemente 20 aufweist, insbesondere drei oder vier. Die Winkelpositionen der Quetschelemente zueinander, also die zwischen benachbarten Quetschelementen befindlichen Winkel, betragen außerhalb des Bereichs der Vorkompression vorzugsweise im Falle von zwei Quetschelementen 180°, im Falle von drei Quetschelementen 120° und Falle von vier Quetschelementen 90°.

Die Quetschelemente können auch unmittelbar am Rotor ausgebildet sein, insbesondere einstückig mit dem Rotor. Sie können alternativ auf Rotorarmen angeordnet sein. Diese sind vorzugsweise gegenüber dem Rotor in umfänglicher Richtung verschwenkbar ausgebildet, so dass über ein Verschwenken in Umfangsrichtung die Vorkompression erzielt werden kann. Die Quetschelemente können insbesondere als Quetschrollen oder Andruckrollen, die in vorteilhafter Weise materialschonend an der Fluidleitung abrollen, oder Gleitschuhe, die sich gleitend über die Fluidleitung bewegen, ausgebildet sein.

Die Erfindung schafft somit Peristaltikpumpe, insbesondere zum Fördern von Fluid in einer Vorrichtung zur extrakorporalen Blutbehandlung, mit einem Pumpengehäuse, in dem ein um eine Rotorachse rotierbarer Rotor mit zumindest zwei in Umfangsrichtung zueinander versetzten Quetschelementen aufgenommen ist und welches eine sich bogenförmig um die Rotorachse erstreckende und radial von dem Rotor beabstandete Stützfläche aufweist, die zum Abstützen eines radial zwischen den Rotor und die Stützfläche einbringbaren Schlauchsegments eingerichtet ist. Zur Sicherstellung eines konstanten Fördervolumens bei gleichzeitiger weitestgehender Schonung des Schlauchsegments, hat die Peristaltikpumpe mit dem Rotor mitdrehende Führungsflächen, die jeweils zu beiden Seiten des Quetschelements in Umfangsrichtung benachbart derart angeordnet und gestaltet sind, dass sie mit der Stützfläche jeweils einen kreissegmentartigen Führungskanal ausbilden, in dem das Schlauchsegment mit vorgegebener Spielpassung radial fixierbar ist.

### Bezugszeichenliste

- A: Rotorachse
- WZS: Zentriwinkel Stützfläche
- WZF: Zentriwinkel Führungsfläche
- PP: Hochdruckseite
- PN: Niederdruckseite
- D: Drehrichtung
- FK1: Führungskonturbereich
- FK2: Führungskonturbereich

- 5: Pumpengehäuse
- 10: Rotor
- 20: Quetschelement
- 30: Stützfläche
- 40: Rotor-Grundkörper
- 41: Schwinge
- 42: Rotorabdeckung
- 44: Taste
- 46: Führungsfläche
- WZ46: Zentriwinkel von 46
- 48: Führungsfläche
- WZ48: Zentriwinkel von 48
- WZG: Gesamt-Zentriwinkel von 46 und 48
- 52: Sockel
- 60: Pumpensegment
- 70: Freiraum

- 310: Rotor
- 320: Quetschelemente
- 330: Stützfläche
- 340: Führungsfläche
- 360: Pumpensegment

## Patentansprüche

1. Peristaltikpumpe, insbesondere zum Fördern von Fluid in einer Vorrichtung zur extrakorporalen Blutbehandlung, mit
einem Pumpengehäuse (5), in dem ein um eine Rotorachse (A) rotierbarer Rotor (10) mit zumindest zwei in Umfangsrichtung zueinander versetzten Quetschelementen (20) aufgenommen ist und welches eine sich bogenförmig um die Rotorachse (A) erstreckende und radial von dem Rotor (10) beabstandete Stützfläche (30) aufweist, die zum Abstützen eines radial zwischen den Rotor (10) und die Stützfläche (30) einbringbaren Schlauchsegments (60) eingerichtet ist, **gekennzeichnet durch**
mit dem Rotor (10) mitdrehende Führungsflächen (46, 48), die jeweils zu beiden Seiten eines Quetschelements (20) in Umfangsrichtung benachbart derart angeordnet und gestaltet sind, dass sie mit der Stützfläche (30) jeweils einen kreissegmentartigen Führungskanal (FK1, FK2) ausbilden, in dem das Schlauchsegment (60) mit vorgegebener Spielpassung radial fixierbar ist.

2. Peristaltikpumpe nach Anspruch 1, **dadurch gekennzeichnet, dass** die einem Quetschelement (20) zugeordneten und diesem benachbarten Führungsflächen (46, 48) jeweils Teilsegmente eines Kreises beschreiben.

3. Peristaltikpumpe nach Anspruch 1 oder 2, **dadurch gekennzeichnet, dass** sich die einem Quetschelement (20) zugeordneten Führungsflächen (46, 48) über einen Gesamt-Zentriwinkel (WZG) erstrecken, der so bemessen ist, dass zwischen Führungsflächen (46, 48) benachbarter Quetschelemente (20) ein sich in Umfangsrichtung erstreckender Freiraum (70) belassen wird.

4. Peristaltikpumpe nach Anspruch 3, **dadurch gekennzeichnet, dass** der Freiraum (70) als seitlicher Zugang für eine Entriegelungstaste (44) nutzbar ist.

5. Peristaltikpumpe nach einem der Ansprüche 1 bis 4, **dadurch gekennzeichnet, dass** sich die einem Quetschelement (20) zugeordneten Führungsflächen (46, 48) über unterschiedlich große Zentriwinkel (WZ46, WZ48) erstrecken.

6. Peristaltikpumpe nach einem der Ansprüche 1 bis 5, **dadurch gekennzeichnet, dass** die Führungsflächen (46, 48) an einer Rotorabdeckung (42) ausgebildet oder an dieser optional auswechselbar befestigt sind.

7. Peristaltikpumpe nach einem der Ansprüche 1 bis 6, bei der die Quetschelemente (20) diametral zueinander versetzt angeordnet sind, **dadurch gekennzeichnet, dass** jeweils zwei Führungsflächen (46, 48) rotationssymmetrisch zueinander ausgebildet sind.

8. Peristaltikpumpe nach einem der Ansprüche 1 bis 7, **dadurch gekennzeichnet, dass** die Führungsflächen (46, 48) von einem Werkstoff gebildet und/oder beschichtet sind, der abriebminimierende Gleiteigenschaften hat.

## Claims

1. A peristaltic pump, in particular for conveying fluid in a device for extracorporeal blood treatment, with
a pump housing (5), in which a rotor (10) rotatable about a rotor axis (A) with at least two squeeze elements (20) offset in a circumferential direction to each other is accommodated and which has a support surface (30) extending arc-shaped about the rotor axis (A) and spaced radially from the rotor (10), wherein the support surface is configured to support a tube segment (60) which can be inserted radially between the rotor (10) and the support surface (30), **characterized by**
co-rotating guide surfaces (46, 48) with the rotor (10), which are arranged and configured adjacent to both sides of a squeeze element (20) in the circumferential direction in such a way that they each form a circular segment-like guiding passage (FK1, FK2) with the support surface (30), in which the tube segment (60) can be fixed radially with a predetermined clearance fit.

2. The peristaltic pump according to claim 1, **characterized in that** the guide surfaces (46, 48) assigned to a squeeze element (20) and adjacent to it each describe partial segments of a circle.

3. The peristaltic pump according to claim 1 or 2, **characterized in that** the guide surfaces (46, 48) assigned to a squeeze element (20) extend over a total centering angle (WZG) that is dimensioned such that a free space or clearance (70) extending in the circumferential direction is left between guide surfaces (46, 48) of adjacent squeeze elements (20).

4. The peristaltic pump according to claim 3, **characterized in that** the clearance (70) can be used as lateral access for an unlocking button (44).

5. The peristaltic pump according to one of claims 1 to 4, **characterized in that** the guide surfaces (46, 48) assigned to a squeeze element (20) extend over central angles (WZ46, WZ48) of different sizes.

6. The peristaltic pump according to one of claims 1 to 5, **characterized in that** the guide surfaces (46, 48) are formed on a rotor cover (42) or are optionally attached to it in a replaceable manner.

7. The peristaltic pump according to one of claims 1 to 6, in which the squeeze elements (20) are arranged diametrically offset to each other, **characterized in that** two guide surfaces (46, 48) are each configured to be rotationally symmetrical to each other.

8. The peristaltic pump according to one of claims 1 to 7, **characterized in that** the guide surfaces (46, 48) are made of and/or coated with a material that has abrasion-minimizing sliding properties.

## Revendications

1. Pompe péristaltique, en particulier pour transporter du fluide dans un dispositif de traitement sanguin extracorporel, avec
un boîtier de pompe (5), dans lequel est logé un rotor (10) pouvant tourner autour d'un axe de rotor (A) avec au moins deux éléments de pressage (20) décalés l'un par rapport à l'autre en direction circonférentielle et qui présente une surface d'appui (30) s'étendant en forme d'arc autour de l'axe de rotor (A) et espacée radialement du rotor (10), surface d'appui qui est configurée pour soutenir un segment de tuyau (60) pouvant être introduit radialement entre le rotor (10) et la surface d'appui (30), **caractérisée en ce que**
des surfaces de guidage (46, 48) tournant avec le rotor (10), qui sont disposées et conçues de manière adjacente en direction circonférentielle, respectivement des deux côtés d'un élément de pressage (20), de sorte qu'elles forment avec la surface d'appui (30) respectivement un canal de guidage (FK1, FK2) en forme de segment de cercle, canal dans lequel le segment de tuyau (60) peut être fixé radialement avec un ajustement de jeu prédéterminé.

2. Pompe péristaltique selon la revendication 1, **caractérisée en ce que** les surfaces de guidage (46, 48) associées à un élément de pressage (20) et adjacentes à celui-ci décrivent respectivement des segments partiels d'un cercle.

3. Pompe péristaltique selon la revendication 1 ou 2, **caractérisée en ce que** les surfaces de guidage (46, 48) associées à un élément de pressage (20) s'étendent sur un angle au centre total (WZG) qui est dimensionné de sorte qu'un espace libre (70) s'étendant en direction circonférentielle est laissé entre des surfaces de guidage (46, 48) d'éléments de pressage (20) adjacents.

4. Pompe péristaltique selon la revendication 3, **caractérisée en ce que** l'espace libre (70) peut être utilisé comme accès latéral pour une touche de déverrouillage (44).

5. Pompe péristaltique selon l'une quelconque des revendications 1 à 4, **caractérisée en ce que** les surfaces de guidage (46, 48) associées à un élément de pressage (20) s'étendent sur des angles au centre (WZ46, WZ48) de tailles différentes.

6. Pompe péristaltique selon l'une quelconque des revendications 1 à 5, **caractérisée en ce que** les surfaces de guidage (46, 48) sont formées sur un couvercle de rotor (42) ou fixées à celui-ci facultativement de manière interchangeable.

7. Pompe péristaltique selon l'une quelconque des revendications 1 à 6, dans laquelle les éléments de pressage (20) sont décalés diamétralement l'un par rapport à l'autre, **caractérisée en ce que** respectivement deux surfaces de guidage (46, 48) sont conçues à symétrie de rotation l'une par rapport à l'autre.

8. Pompe péristaltique selon l'une quelconque des revendications 1 à 7, **caractérisée en ce que** les surfaces de guidage (46, 48) sont formées et/ou revêtues d'un matériau qui présente des propriétés de glissement minimisant l'abrasion.
